# EUROPEAN PATENT SPECIFICATION

(11) **EP 2 167 062 B1**
(45) Date of publication and mention of the grant of the patent: **18.02.2015**
(21) Application number: 08760860.0
(22) Date of filing: 11.06.2008
(51) Int. Cl.: A61K 31/12, A61K 31/203, A61K 45/06, A61P 17/10, A61Q 5/00, A61K 8/04, A61Q 19/00

(54) **Compositions and uses comprising a retinoid compound, an anti-irritant compound and benzoyl peroxide in acne**
Zusammensetzungen und Verwendungen mit einer Retinoid-Verbindung, einer nichtreizenden Verbindung und Benzoylperoxid bei Akne
Compositions comprenant un composé rétinoïde, un composé anti-irritant et du peroxyde de benzoyle et utilisations dans le traitement de l'acné

(30) Priority: 11.06.2007 FR 0755658; 18.06.2007 US 929205 P
(43) Date of publication of application: 31.03.2010
(73) Proprietor: Galderma Research & Development, 06410 Biot (FR)
(72) Inventor: MALLARD, Claire, F-06250 Mougins (FR)
(74) Representative: Casalonga
(86) International application number: PCT/EP2008/057310
(87) International publication number: WO 2008/152064

(56) References cited:
- EP-A- 1 586 308
- WO-A-97/48371
- WO-A-03/055472
- WO-A-2006/070093
- GB-A- 2 130 486
- US-A- 4 189 501
- KORKUT C ET AL: "BENZOYL PEROXIDE, ADAPALENE, AND THEIR COMBINATION IN THE TREATMENT OF ACNE VULGARIS" JOURNAL OF DERMATOLOGY, JAPANESE DERMATOLOGICAL ASSOCIATION, TOKYO, JP, vol. 32, no. 3, 1 January 2005 (2005-01-01), pages 169-173, XP009075640 ISSN: 0385-2407
- BERSON D ET AL: "Management of Acne" JOURNAL OF THE AMERICAN ACADEMY OF DERMATOLOGY, C.V. MOSBY, ST. LOUIS, MO, vol. 49, 2003, pages S1-S37, XP002365975 ISSN: 0190-9622
- CAPIZZI ET AL: "SKIN TOLERABILITY AND EFFICACY OF COMBINATION THERAPY WITH HYDROGEN PEROXIDE STABILIZED CREAM AND ADAPALENE GEL IN COMPARISON WITH BENZOYL PEROXIDE CREAM AND ADAPALENE GEL IN COMMON ACNE. A RANDOMIZED, INVESTIGATOR-MASKED, CONTROLLED TRIAL" BRITISH JOURNAL OF DERMATOLOGY, XX, XX, vol. 151, no. 2, 2004, pages 481-484, XP008073258 ISSN: 0007-0963
- EGGENSPERGER H ET AL: "Glycyrrhetinsäure - und was man von einem multiaktiven Wirkstoff für die Kosmetik und Dermatologie erwarten darf//Glycyrrhetinic acid. Expectations from a multi - active substance for the cosmetics and dermatology" SOFW-JOURNAL SEIFEN, OELE, FETTE, WACHSE, VERLAG FUR CHEMISCHE INDUSTRIE, AUGSBURG, DE, vol. 122, no. 14, 1 January 1996 (1996-01-01), pages 990-997, XP002257130 ISSN: 0942-7694

## Description

The present invention relates to compositions for topical application, and to the uses thereof as cosmetic or pharmaceutical products, said compositions being for use in the treatment of dermatological disorders, and in particular in the treatment of acne.

Acne is a common multi-factor pathology that attacks skin rich in sebaceous glands (face, shoulder area, arms and intertriginal areas). It is the most commonly occurring form of dermatosis. The following five pathogenic factors play a determining role in the formation of acne:
1. genetic predisposition;
2. overproduction of sebum (seborrhoea);
3. androgens;
4. follicular keratinization disorders (comedogenesis); and
5. bacterial colonization and inflammatory factors.

There are several forms of acne, the common factor of all being attack of the pilosebaceous follicles. Mention may be made in particular of acne conglobata, cheloid acne of the nape of the neck, acne medicamentosa, recurrent miliary acne, necrotic acne, neonatal acne, premenstrual acne, occupational acne, acne rosacea, senile acne, solar acne and common acne.

Common acne, also known as polymorphic juvenile acne, is the most common. It comprises four stages:
- stage 1 corresponds to comedonic acne characterized by a large number of open and/or closed comedones and of microcysts;
- stage 2, or papulopustular acne, is of mild to moderate seriousness. It is characterized by the presence of open and/or closed comedones, of microcysts, but also of red papules and pustules. It mainly affects the face and leaves few scars;
- stage 3, or papulocomedonic acne, is more serious and extends to the back, the chest and the shoulders. It is accompanied by a large number of scars;
- stage 4, or nodulocystic acne, is accompanied by numerous scars. It exhibits nodules and also painful voluminous crimson pustules.

The various forms of acne described above can be treated with active agents such as anti-seborrheic agents and anti-infectives, for example benzoyl peroxide (in particular the product Eclaran^{®} sold by the company Pierre Fabre), with retinoids such as tretinoin (in particular the product Retacnyl^{®} sold by the company Galderma) or isotretinoin (the product Roaccutane^{®} sold by Laboratoires Roche), or else with naphthoic acid derivatives. Naphthoic acid derivatives such as, in particular, 6-[3-(1-adamantyl)-4-methoxyphenyl]-2-naphthoic acid, which is commonly called adapalene (the product Differine^{®} sold by the company Galderma), are widely described and recognized as active ingredients that are just as effective as tretinoin for the treatment of acne.

However, in order to increase the effectiveness of treatments, especially treatments for dermatological disorders, and in particular for acne, and to reduce the toxicity of the active ingredients (Cunliffe W.J., J. Dermatol. Treat., 2000, 11 (suppl2), S13-S14), use is commonly made of several categories of active ingredients.

An article by Korkut and Piskin, J. Dermatology, 2005, 32: 169-173, reports the results of a study comparing a treatment combining application of adapalene in the evening and application of BPO in the morning, relative to an application of each of the active principles alone. The authors do not observe any superiority of the combined treatment over a period of 11 weeks of treatment.

Since the multiple application of various dermatological products is quite laborious and demanding for the patient, the value of seeking to obtain a new treatment which is effective on dermatological conditions, in particular acne, in a stable composition which offers good cosmeticity, which significantly improves tolerance and which makes it possible to increase patient compliance, can therefore be understood.

Combinations of active agents are now beginning to appear. Mention may thus be made of the DUAC combination comprising clindamycin and benzoyl peroxide or combinations of antibiotics. Among these, mention may also be made of a gel comprising at least one retinoid and benzoyl peroxide as described in application WO 03/55472.

However, the formulation of such a composition comprising several active agents, including benzoyl peroxide, poses several problems.

First of all, the effectiveness of the benzoyl peroxide is linked to its decomposition when it is brought into contact with the skin. It is the oxidizing properties of the free radicals produced during this decomposition which produces the desired effect. Thus, in order to maintain optimum effectiveness for the benzoyl peroxide, it is important to prevent its decomposition before use, i.e. during storage.

Now, benzoyl peroxide is a chemical compound that is unstable, which makes it difficult to formulate it in finished products.

The solubility and the stability of benzoyl peroxide have been studied in ethanol, propylene glycol and various mixtures of polyethylene glycol 400 (PEG 400) and water (Chellquist E.M. and Gorman W.G., Pharm. Res., 1992, Vol 9: 1341-1346). The authors thus note that benzoyl peroxide in solution degrades more or less rapidly in all the solvents studied, depending on the type of solvent and on its concentration.

The benzoyl peroxide degradation times observed are so short that they do not make it possible to prepare a product that is intended to be sold.

It is known, moreover, that benzoyl peroxide is more stable in water and in propylene glycol when it is in suspension (i.e. in disperse form), since it is not degraded after 90 days of storage in these solvents.

Thus, in order to limit the problem of rapid instability of benzoyl peroxide in solution, it has been found to be advantageous to formulate benzoyl peroxide in dispersed form.

Another difficulty to be overcome in the preparation of a composition comprising both a retinoid, an anti-irritant and benzoyl peroxide is that most retinoids are particularly sensitive to natural oxidation, to visible light and to ultraviolet radiation. Since benzoyl peroxide is a strong oxidizing agent, the chemical compatibility of these compounds in the same formulation poses many problems of stability from the physical and chemical point of view.

In addition, it has been reported that benzoyl peroxide can sometimes induce dryness of the skin and on certain occasions irritation of the skin.

Among the retinoids commonly used, adapalene in particular exhibits a unanimously proven effectiveness. However, it would be advantageous and useful to reduce the irritation caused by retinoids applied topically, including adapalene, although its tolerance is greater than that of its competitors belonging to the same chemical category (tretinoin, tazarotene).

The term "irritation" is intended to mean in particular the symptoms or the conditions linked to the application to the skin of chemical products of natural or synthetic origin, used in cosmetics or dermatology, and which can be reflected in particular by an inflammation, an erythema, an oedema, redness, itching, pain, burning, a sting, or else tingling.

Given the above, a problem that the invention proposes to solve is that of producing a stable topical composition that is barely irritant or not at all, comprising, in a pharmaceutically acceptable medium, a retinoid, benzoyl peroxide and an anti-irritant, for the treatment of dermatological disorders, and in particular for the treatment of acne.

The presence of an anti-irritant makes it possible to significantly improve the tolerance of the composition comprising a retinoid and benzoyl peroxide, and therefore to overcome the problem of irritation. Advantageously, such a composition according to the invention makes it possible to increase the concentrations of the active ingredients while at the same time limiting their side effects. In addition, when said composition is in the form of a gel, a cream-gel or an emulsion, it provides emollience and avoids in particular leaving too greasy a feel on the skin.

In addition, the pharmaceutical or cosmetic compositions according to the invention conserve, throughout their Shelf life, precise physicochemical criteria for guaranteeing their pharmaceutical or cosmetic quality. Among these criteria, it is necessary for the rheological properties to be conserved. These rheological properties define the behaviour and the texture of the composition during application, but also the properties of release of the active ingredients.

Now, the applicant has produced a composition which meets these needs while at the same time thus overcoming the problem of irritation.

A first subject of the solution as proposed by the invention is thus a composition for topical application comprising, in a physiologically acceptable medium, at least one retinoid compound in dispersed form chosen from all-trans retinoic acid, isotretinoin, motretinide, and naphthoic acid derivatives of formula (I), and salts and esters thereof: 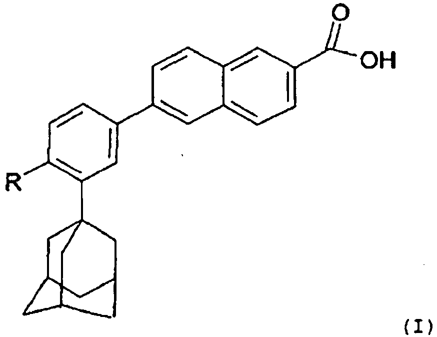 where R represents a hydrogen atom, a hydroxyl radical, a branched or nonbranched alkyl radical containing from 1 to 4 carbon atoms, an alkoxy radical containing from 1 to 10 carbon atoms or a cycloaliphatic radical which is substituted or unsubstituted, at least one wetting agent, at least one anti-irritant compound chosen from 18β-glycyrrhetinic acid (enoxolone), its salts and its derivatives as described in the following pages, and benzoyl peroxide in dispersed form.

In a particular embodiment of the invention, the retinoid compound is a naphthoic acid derivatives of formula (I), and salts and esters thereof.

In a specific embodiment, the naphtoic acid of formula (I) is characterized in that the alkyl radical is the methyl, ethyl, propyl or butyl radical; the alkoxy radical is the methoxy, ethoxy, propoxy, butoxy, hexyloxy or decyloxy radical; and the cycloaliphatic radical is the 1-methylcyclohexyl radical or the 1-adamantyl radical.

In a preferred embodiment, the retinoid compound is chosen from 6-[3-(1-adamantyl)-4-methoxyphenyl]-2-naphthoic acid (adapalene), 6-[3-(1-adamantyl)-4-hydroxyphenyl]-2-naphthoic acid, 6-[3-(1-adamantyl)-4-decyloxyphenyl]-2-naphthoic acid and 6-[3-(1-adamantyl)-4-hexyloxyphenyl]-2-naphthoic acid and salts and esters thereof. More preferably, the retinoid compound is 6-[3-(1-adamantyl)-4-methoxyphenyl]-2-naphthoic acid (adapalene) and salts and esters thereof.

In a specific embodiment, the concentration of retinoid compound is between 0.001% and 10%, preferentially between 0.01% and 5%, preferably between 0.01% and 1%, more preferentially between 0.01% and 0.5%, and preferentially between 0.1% and 0.3% by weight of the total weight of the composition. More preferred, the concentration of retinoid compound is equal to 0.1% or equal to 0.3%.

The anti-irritant compound's salt or derivative is chosen from the potassium salt of 18β-glycyrrhetinic acid, the sodium salt of 18β-glycyrrhetinic acid, the monoammonium salt of 18β-glycyrrhetinic acid, the disodium succinate salt of 18β-glycyrrhetinic acid, the dipotassium salt of 18β-glycyrrhetinic acid and the monoester of 18β-glycyrrhetinic acid.

Preferably, the concentration of anti-irritant compound is between 0.01% and 10%, preferentially between 0.1% and 7%.

The benzoyl peroxide is in dispersed form in the composition. In general, the benzoyl peroxide could be in encapsulated or free form. Preferably, the composition comprises between 0.0001% and 20% of benzoyl peroxide, preferentially between 0.025% and 10%, even more preferentially between 2.5% and 5%.

The composition is for topical application. Preferably, the composition is in the form of aqueous, aqueous-alcoholic or oily dispersions, dispersions of the lotion type, aqueous, anhydrous or lipophilic gels, emulsions of liquid or semi-liquid consistency of the milk type, obtained by dispersion of a fatty phase in an aqueous phase (O/W) or vice versa (W/O), or suspensions or emulsions of soft, semi-liquid or solid consistency of the cream, gel, cream-gel, foam or ointment type, or microemulsions, microcapsules, microparticles or vesicular dispersions of ionic and/or nonionic type, in the form of sprays, or else in the form of dermal devices such as patches.

More preferably, the composition is in the form of a gel, a cream-gel or an emulsion.

Most preferred, said composition is a medicament.

A second subject of the present invention is the use of a composition according to the invention in the preparation of a medicament for use in the treatment and/or prevention of dermatological conditions linked to a keratinization disorder relating to cell differentiation and proliferation, in particular for treating common acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and acne medicamentosa. Preferably, the preparation of a pharmaceutical composition is intended for use in preventing, inhibiting or treating common acne.

A subject of the present invention is a method for preparing a composition according to the invention, characterized in that it comprises a step of mixing at least one retinoid compound with benzoyl peroxide and with at least one anti-irritant compound and in particular in the form of a gel, and also a method for preparing a composition in the form of a cream-gel and/or a method for preparing a composition in the form of an emulsion.

The invention also provides a method for treating and/or preventing and/or inhibiting dermatological conditions linked to a keratinization disorder relating to cell differentiation and proliferation, in particular for treating common acne, comedonic acne, papulopustular acne, papulocomedonic acne, nodulocystic acne, acne conglobata, cheloid acne of the nape and/or the neck, recurrent miliary acne, necrotic acne, neonatal acne, occupational acne, acne rosacea, senile acne, solar acne and acne medicamentosa, comprising administering to an individual in need thereof, a therapeutical effective amount of composition defined previously.

Finally, a subject of the present invention is also a nontherapeutic cosmetic treatment process for embellishing the skin or its surface appearance, in which a composition according to the invention is applied to the skin and/or its integument annexes. In a preferred embodiment, the treatment of skin is for skin with an acneic tendency or for combating the greasy appearance of the skin.

Throughout the present text, unless otherwise specified, it is understood that, when concentration ranges are given, they include the upper and lower limits of said range. Similarly, unless otherwise indicated, the proportions of the various constituents of the composition are expressed as percentage by weight (m/m) of the total weight of said composition.

According to the invention, the composition comprises, in a physiologically acceptable medium, at least one compound of retinoid type, at least one anti-irritant compound and benzoyl peroxide, and is further subject to the limitations described previously.

The term "physiologically acceptable medium" is intended to mean a medium compatible with the skin, the mucous membranes and/or the appendages.

The retinoid compound according to the invention may be chosen from all-trans retinoic acid (or tretinoin), isotretinoin or else motretinide.

The retinoid compound according to the invention is preferably chosen from naphthoic acid derivatives of formula (I), and salts and esters thereof: 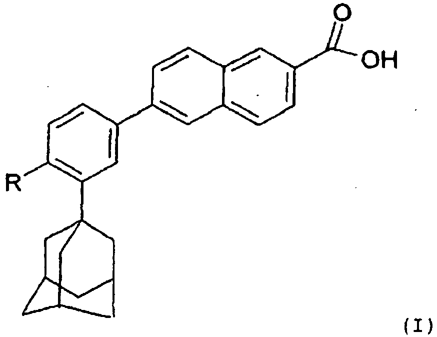 where R represents a hydrogen atom, a hydroxyl radical, a branched or nonbranched alkyl radical containing from 1 to 4 carbon atoms, an alkoxy radical containing from 1 to 10 carbon atoms or a cycloaliphatic radical which is substituted or unsubstituted.

The expression "linear or branched alkyl radical containing from 1 to 4 carbon atoms" is intended to mean preferably methyl, ethyl, propyl and butyl radicals.

The expression "alkoxy radical containing from 1 to 10 carbon atoms" is intended to mean preferably methoxy, ethoxy, propoxy, butoxy, hexyloxy and decyloxy radicals.

The term "cycloaliphatic radical" is intended to mean preferably monocyclic or polycyclic radicals, such as the 1-methylcyclohexyl radical or the 1-adamantyl radical.

The term "salts of the naphthoic acid derivatives" is intended to mean salts formed with a pharmaceutically acceptable base, in particular an inorganic base such as sodium hydroxide, potassium hydroxide or aqueous ammonia, or an organic base such as lysine, arginine or N-methylglucamine, but also the salts formed with fatty amines such as dioctylamine, aminomethyl propanol and stearylamine.

The term "esters of the naphthoic acid derivatives" is intended to mean esters formed with pharmaceutically acceptable alcohols.

Preferably, among the naphthoic acid derivatives that may be part of the compositions according to the invention, 6-[3-(1-adamantyl)-4-methoxyphenyl]-2-naphthoic acid (adapalene), 6-[3-(1-adamantyl)-4-hydroxyphenyl]-2-naphthoic acid, 6-[3-(1-adamantyl)-4-decyloxyphenyl]-2-naphthoic acid or 6-[3-(1-adamantyl)-4-hexyloxyphenyl]-2-naphthoic acid will be chosen.

Even more preferably, the retinoid compound that can be used according to the invention is chosen from adapalene (6-[3-(1-adamantyl)-4-methoxyphenyl]-2-naphthoic acid), salts thereof and esters thereof.

The term "adapalene salts" is intended to mean in particular the salts formed with a pharmaceutically acceptable base, in particular inorganic bases such as sodium hydroxide, potassium hydroxide or aqueous ammonia, or organic bases such as lysine, arginine or N-methylglucamine.

The term "adapalene salts" is also intended to mean the salts formed with fatty amines such as dioctylamine, aminomethyl propanol and stearylamine.

Preferably, the retinoid compound according to the invention is adapalene.

Advantageously, the composition according to the invention does not comprise any depigmenting agent distinct from the retinoid compound, in particular adapalene.

According to a specific embodiment of the invention, the adapalene is in dispersed form in the composition.

In the compositions according to the invention, the concentration of retinoid compound is between 0.0001% and 10%, in particular between 0.10% and 5%, preferably between 0.01% and 1%, more preferentially between 0.01% and 0.5%, and preferentially between 0.1% and 0.3% by weight of the total weight of the composition.

Even more preferentially, the concentration of retinoid compound is equal to 0.1%. Alternatively, the concentration of retinoid compound is preferably equal to 0.3%.

According to the invention, the term "anti-irritant" is intended to mean an active agent that modulates the manifestations of sensitive skin, i.e. the manifestations of skin irritation, such as stinging, tight skin, burning sensation and redness, and is selected from the glycyrrhetinic acid compounds described previously, and in the following.

The expression "sensitive skin" covers both irritable and/or reactive skin and intolerant skin.

Irritable and/or reactive skin is skin which reacts through pruritis, i.e. through itching or through stinging, to various factors such as the environment, emotions, foods, wind, friction, shaving, soap, surfactants, hard water with a high calcium content, temperature variations or wool. In general, these signs are associated with dry skin with or without dry patches, or with skin that exhibits erythema.

Intolerant skin is skin which reacts through sensations of burning, tighting, stinging and/or redness, to various factors such as the environment, emotions, foods and certain cosmetic products. In general, these signs are associated with hyperseborrheic or acneic skin with or without dry patches and associated with erythema.

The use of these specific anti-irritants makes it possible to reduce the irritation caused by the active ingredients, in particular the retinoids.

The anti-irritants that are used according to the present invention are chosen from 18β-glycyrrhetinic acid (enoxolone), its salts and its derivatives.

Salts and derivatives are selected from the potassium salt of 18β-glycyrrhetinic acid, the sodium salt of 18β-glycyrrhetinic acid, the monoammonium salt of 18β-glycyrrhetinic acid (ammonium glycyrrhetinate), the disodium succinate salt of 18β-glycyrrhetinic acid, the dipotassium salt of 18β-glycyrrhetinic acid, and the monoester of 18β-glycyrrhetinic acid.

Preferably, the anti-irritant is the potassium salt of 18β-glycyrrhetinic acid.

The anti-irritant used according to the invention may be of natural or synthetic origin.

The term "natural origin" is intended to mean an anti-irritant in the pure state or in solution irrespective of its concentration in said solution, obtained, by various methods, from a natural element.

The term "synthetic origin" is intended to mean an anti-irritant in the pure state or in solution, irrespective of its concentration in said solution, obtained by chemical synthesis.

The concentration of anti-irritant compound used according to the invention is, for its part, between 0.01% and 10%, preferentially between 0.1% and 7%.

The composition according to the invention also comprises benzoyl peroxide in dispersed form.

Benzoyl peroxide can in general also be used in free form or else in an encapsulated form, for example in a form adsorbed onto, or absorbed into, any porous support. It may, for example, be encapsulated in a polymeric system consisting of porous microspheres, for instance microsponges sold under the name Microsponges P009A Benzoyl peroxide^{™} by the company Amcol.

Advantageously, the particle size of the benzoyl peroxide is such that at least 80% by number of the particles, preferably at least 90% by number of the particles, have a diameter of less than 25 µm and at least 99% by number of the particles have a diameter of less than 100 µm.

The concentration of benzoyl peroxide used in the compositions according to the invention is between 0.0001% and 20%, preferentially between 0.025% and 10%, even more preferentially between 0.5% and 5% and more preferred 2.5% and 5%.

The composition according to the invention may also in particular comprise at least one propenetrating agent.

The concentration of propenetrating agents used in the compositions according to the invention is between 0.0001% and 20%.

The propenetrating agents should generally not solubilize the active agents at the percentage used, not cause exothermic reactions harmful to the benzoyl peroxide, aid good dispersion of the active agents and have antifoam properties.

The composition according to the invention may also in particular comprise at least one pH-independent gelling agent.

The term "pH-independent gelling agent" is intended to mean a gelling agent capable of conferring a sufficient viscosity on the composition so as to maintain both the retinoid, the anti-irritant and the benzoyl peroxide in suspension, even under the influence of a variation in pH due to the release of benzoic acid by the benzoyl peroxide. The gelling agent according to the invention also has good physical stability, i.e. no decrease in viscosity is observed over time at temperatures between 4 and 40°C, maintaining good chemical stability of the active agents, i.e. no degradation of the active agents is observed over time and at temperatures between 4 and 40°C.

By way of nonlimiting examples of gelling agents and/or suspending agents and/or pH-independent agents that can be part of the compositions according to the invention, mention may be made of the microcrystalline cellulose et sodium carboxymethyl cellulose (such as this sold as Avicel CL-611 or RC/CL by FMC Biopolymer company), the "electrolyte-insensitive" carbomers sold under the name Ultrez 20^{™}, Carbopol 1382^{™}, Pemulen TR1, Pemulen TR2 or Carbopol ETD2020^{™} by the company Noveon; polysaccharides, nonlimiting examples of which include xanthan gum, such as Xanturall80^{™} sold by the company Kelco, guar gum, chitosans, carrageenans, cellulose and its derivatives such as hydroxypropylmethylcellulose, in particular the product sold under the name Methocel E4 premium^{™} by the company Dow Chemical or hydroxyethylcellulose, in particular the product sold under the name Natrosol HHX 250^{™} by the company Aqualon, the family of magnesium aluminium silicates such as Veegum K^{™} sold by the company Vanderbilt, the family of carrageenans in particular those in the four following sub families: k, λ, β, ω such as Viscarin ® or Gelcarins ® sold by the company IMCD, the family of acrylic polymers coupled to hydrophobic chains, such as the PEG-150/decyl/SMDI copolymer sold under the name Aculyn 44^{™} (polycondensate comprising at least, as elements, a polyethylene glycol comprising 150 or 180 mol of ethylene oxide, decyl alcohol and methylene-bis(4-cyclohexylisocyanate) (SMDI), at 35% by weight in a mixture of propylene glycol (39%) and water (26%)), the family of modified starches such as the modified potato starch sold under the name Structure Solanace^{™}, or else mixtures thereof, and the gelling agents of the polyacrylamide family, such as the sodium acryloyl-dimethyltaurate copolymer/isohexadecane/polysorbate 80 mixture sold under the name Simulgel 600PHA^{™} by the company Seppic, or the polyacrylamide/isoparaffin C13-14/laureth-7 mixture such as, for example, that sold under the name Sepigel 305^{™} by the company Seppic.

The preferred gelling agents are derived from the polyacrylamide family, such as Simulgel 600PHA^{™} or Sepigel 305^{™}; "electrolyte-insensitive" carbomers such as Carbopol 1382^{™}; polysaccharides such as xanthan gum; cellulose derivatives such as hydroxypropylmethylcellulose or hydroxyethylcellulose; and magnesium aluminium silicates, alone or as a mixture.

The pH-independent gelling agent as described above can be used at the preferential concentrations ranging from 0.001% to 15% and more preferentially between 0.15% and 5%.

The composition according to the invention comprises at least one wetting agent.

The wetting capacity is the tendency of a liquid to spread out over a surface.

Preferably, they are wetting agents which have an HLB (hydrophilic/lipophilic balance) of 7 to 18, or nonionic wetting agents of polyoxyethylenated and/or polyoxypropylenated copolymer type. As non limiting examples of wetting agents, mention can be made of Poloxamers and more particularly the product as known as Synperonic PE/L44 (Polyethylene-polypropylene glycol; Polyoxyethylene-Polyoxypropylene Block Copolymer)and/or Synperonic PE/L62 sold by the company Uniqema, glycols such as those known as propylene glycol, dipropylene glycol, lauroglycol, propylene glycol dipelargonate, ethoxydiglycol. They should be liquid so as to incorporate readily into the composition without it being necessary to heat it.

Among the wetting agents whose role it is to reduce the surface tension and to allow greater spreading of the liquid, use is preferentially made, without this list being limiting, of compounds such as those of the poloxamers and/or glycols families and more particularly Synperonic PE/L44 and/or Synperonic PE/L62 and/or compounds such as propylene glycol, dipropylene glycol, propylene glycol dipelargonate, lauroglycol, ethoxydiglycol.

By way of preferred wetting agent, mention may be made of propylene glycol or synperonic PE//L44 (Poloxamer 124^{™}).

The concentration of wetting agents used in the compositions according to the invention is between 0.0001% and 20%, preferentially between 0.1% and 10% and more preferably between 2 and 7% in weight with regards to the total composition weight.

The composition according to the invention may also in particular comprise at least one emulsifier.

Preferably, the emulsifier used is different from the wetting agents.

The term "emulsifiers" is intended to mean amphiphilic compounds having a hydrophobic part which has an affinity for oil and a hydrophilic part which has an affinity for water, thus creating a link between the two phases. Ionic or nonionic emulsifiers therefore stabilize emulsions (O/W) by adsorbing them into one another at the interface and forming lamellar layers of liquid crystals.

The emulsifying capacity of nonionic emulsifiers is closely linked to the polarity of the molecule. This polarity is defined by the HLB (hydrophilic/lipophilic balance).

A high HLB indicates that the hydrophilic fraction is predominant and, conversely, a low HLB indicates that the lipophilic part is predominant. For example, HLB values of greater than approximately 10 correspond to hydrophilic surfactants.

The emulsifiers may be categorized, according to their structure, under the generic terms "ionic" (anionic, cationic, amphoteric) or "nonionic". The nonionic emulsifiers are emulsifiers which do not dissociate to ions in water and are therefore insensitive to variations in pH.

The nonionic emulsifiers are particularly suitable for the preparation of oil-in-water type emulsions. Thus, the emulsifying system comprises at least one nonionic emulsifier, with a predominant hydrophilic fraction, i.e. having a high HLB, of greater than approximately 10.

By way of examples of nonionic emulsifiers having a high HLB, mention may be made of sorbitan esters such as the POE (20) sorbitan monooleate sold under the name Tween 80^{™} (HLB = 15), or the POE (20) sorbitan monostearate sold under the name Tween 60^{™} (HLB = 14.9), fatty alcohol ethers such as the POE (21) stearyl ether (HLB = 15.5), or the ceteareth 20 sold under the name Eumulgin B2^{™} by Cognis (HLB of 15.5) polyoxyethylene glycol esters such as glyceryl stearate and PEG 100 stearate sold under the trade name Arlacel 165 FL ® (HLB=11) by the company Uniqema , PEG 6 Stearate and PEG 32 stearate sold under the trade name TEFOSE 1500 ® (HLB= 10) by the company Gateffossé, sucroesters with high HLB such as PEG 20 methyl glucose sesquistearate sold under the trade name glucamate SSE20 (HLB=15) by the company Amerchol and sucrose laurate sold under the trade name Surfhope C-1216® (HLB=16) and sucrose stearate sold under the trade name Surfhope C-1811® (HLB=11) by the company Gattefossé.

Preferably, said nonionic emulsifiers with a high HLB have an HLB of between 10 and 18.

As examples of nonionic emulsifiers with a low HLB (lipophilic), mention will be made of sorbitan esters such as sorbitan monostearate (HLB=4.7) (sold under the name Span 60^{™} by Uniqema company), glycerol esters such as glycerol monostearate (sold under the name Cutina GMSVPH^{™} by Cognis company) such as glyceryl monostearate (Cutina GMS^{™} (HLB=3.8) from Cognis company), polyethylene glycol esters such as PEG-6 isostearate sold with the trade name Olépal isostearic (HLB=8) by the company Gattefossé, sucroesters with low HLB such as methyl glucose sesquistearate sold under the trade name Glucate SS (HLB=6) by the company Amerchol and sucrose dilaurate sold under the trade name Surfhope C-1205 (HLB=5) and sucrose tristearate sold under the trade name Surfhope C-1803 (HLB=3) by the company Gattefossé.

Preferably, said nonionic emulsifiers with a low HLB have an HLB of less than 10.

The nonionic emulsifiers may be used alone or as a mixture of two or more of them so as to form the emulsifying system.

Preferably, one or more "nonionic emulsifier with a high HLB"/"nonionic emulsifier with a low HLB" pairs will be used as emulsifying system; it may in particular be a nonionic emulsifying system comprising at least one nonionic emulsifier having an HLB of greater than approximately 10 and at least one nonionic surfactant having an HLB of less than approximately 10.

The ratio of each of the two emulsifiers forming the abovementioned pair is most commonly determined by calculating the required HLB of the fatty phase used.

By way of preferred emulsifiers, mention may be made of hydrophilic emulsifiers of the type glyceryl stearate & PEG-100 stearate sold under the name Arlacel 165FL^{™} by the company Uniqema; the PEG 6 stearate and PEG 32 stearate sold under the name Tefose 1500^{™} by Gattefosse, lipophilic emulsifiers of sucrose ester type, such as the glucate SS^{™} (methyl glucose sesquistearate) and glucamate SSE 20^{™} (PEG 20 methyl glucose sesquistearate) sold by Amerchol, the polyoxyethylene (21) stearyl ether sold under the name Brij721^{™} by the company Uniqema, and the ceteareth 20 sold under the name Eumulgin B2PH^{™} by Cognis.

According to the invention, the preferred concentrations of emulsifiers are between 0.001% and 20%. More preferably, the concentration is between 1% and 15%, and preferably between 3% and 11% by weight, relative to the total weight of the composition.

The composition according to the invention may also in particular comprise at least one chelating agent and/or at least one preservative.

Among the chelating agents, mention may be made, by way of nonlimiting examples, of diethylenetriaminepentaacetic acid (DTPA), ethylenediamine-di-(O-hydroxyphenylacetic) acid (EDDHA), 2-hydroxyethylenediamine-triacetic acid (HEDTA), ethylenediamine-di-(O-hydroxy-p-methylphenyl)acetic acid (EDDHMA), ethylenediaminetetraacetic acid (EDTA) and ethylenediamine-di-(5-carboxy-2-hydroxyphenyl)acetic acid (EDDCHA).

As preferred chelating agent, mention can be made of ethylene diamine tetraacetic acid (EDTA).

Among the preservatives, mention may be made, by way of nonlimiting examples, of benzoic acid and its derivatives such as benzyl alcohol, benzalkonium chloride, sodium benzoate, bronopol, chlorhexidine, chlorocresol and its derivatives, ethyl alcohol, phenethyl alcohol, phenoxyethanol, potassium sorbate, diazolidinylurea, and parabens such as propylparaben or methylparaben, taken alone or as mixtures.

By way of preferred preservative, mention may be made of parabens and phenoxyethanol or benzalkonium chloride, taken alone or as a mixture.

The compositions of the invention may also in particular comprise any additive normally used in the cosmetics or pharmaceutical field, such as neutralizers or pH adjusters such as well known mineral or organic bases or acids, such as example triethanolamine, NaOH 10% solution, sodium succinic acid /succinate buffer, sodium citric acid/citrate buffer, humectants and/or emolients, sunscreens, antioxidants, fillers, electrolytes, dyes, customary inorganic or organic bases or acids, fragrances, essential oils, active cosmetic agents, moisturizers, vitamins, essential fatty acids, sphingolipids, self-tanning compounds such as DHA, and agents for calming and protecting the skin optionally one stabilizer of benzoyl peroxide (such as non limited example sodium docusate, sodium C14-16 olefin sulfonate).

Of course, those skilled in the art will take care to select this or these possible additional compound(s), and/or the amount thereof, in such a way that the advantageous properties of the composition according to the invention are not, or not substantially, impaired.

The concentrations of said additives of the composition are between 0.001% and 20% by weight, relative to the total weight of the composition.

The compositions according to the present invention may be in any of the galenical forms normally used for topical application, in particular in the form of aqueous, aqueous-alcoholic or oily dispersions, dispersions of the lotion type, aqueous, anhydrous or lipophilic gels, emulsions of liquid consistency (in particular 'compatible with a presentation form of impregnated wipe type) or semi-liquid consistency of the milk type, obtained by dispersion of a fatty phase in an aqueous phase, oil-in-water (O/W), or vice versa water-in-oil (W/O), or suspensions or emulsions of soft, semi-liquid or solid consistency, of the cream, cream-gel, foam or ointment type, or microemulsions, microcapsules, microparticles or vesicular dispersions of ionic and/or nonionic type, in the form of sprays, or else in the form of dermal devices such as patches.

The term "topical application" is intended to mean application to the skin or the mucous membranes.

Preferably, the compositions according to the invention are in the form of a gel or a cream-gel of semi-liquid consistency of the milk type to solid consistency of the cream type, obtained by dispersion of a fatty phase in an aqueous phase (O/W).

Preferably, the compositions according to the invention are in the form of an emulsion, preferably a light emulsion in the form of an (O/W) emulsion.

The term "light emulsion" is intended to mean an emulsion containing a low proportion of fatty phase, the aqueous phase remaining predominant.

The term "emulsion" is intended to mean a liquid system comprising two fluids that are insoluble or relatively insoluble in one another, and in which one of the fluids disperses in the other in microscopic particles. Preferably, the emulsions used comprise at least one emulsifier, a polar hydrophilic, preferably aqueous, phase and a nonpolar fatty phase. Preferably, they are in the form of emulsions (O/W or W/O).

In order to obtain this essential stabilization, an emulsifier which reduces the surface tension between the two phases is introduced. The emulsions have an important role in dermatological and cosmetic products because said emulsions correspond to the physiological needs of the skin, and make it possible to bring about uniform penetration of both water-soluble substances and oil-soluble substances.

Those skilled in the art will take care to choose the excipients constituting the compositions according to the invention according to the galenical form desired, and in such a way that the advantageous properties of the composition according to the invention are respected.

The fatty phase of the composition according to the invention may comprise, for example, plant, mineral, animal or synthetic oils, silicone oils, and mixtures thereof.

As an example of a mineral oil, mention may, for example, be made of liquid paraffins of various viscosities, such as Primol 352^{®}, Marcol 82^{®} and Marcol 152^{®} sold by the company Esso.

As a plant oil, mention may be made of sweet almond oil, palm oil, soybean oil, sesame oil and sunflower oil.

As an animal oil, mention may be made of lanolin, squalene, fish oil, and mink oil, with, as a derivative, the squalane sold under the name Cosbiol^{®} by the company Laserson.

As a synthetic oil, mention may be made of an ester such as cetearyl isononanoate, for instance the product sold under the name Cetiol SN PH^{®} by the company Cognis France, diisopropyl adipate, for instance the product sold under the name Crodamol DA^{®} by the company Croda, isopropyl palmitate, for instance the product sold under the name Crodamol IPP^{®} by the company Croda, triglycerides such as caprylic/capric triglyceride, for instance Miglyol 812^{®} sold by the company Hüls/Univar, polymers such as hydrogenated polyisobutene and derivatives.

As a volatile or non-volatile silicone oil, mention may be made of dimethicones, for instance the products sold under the name Dow Corning 200 fluid^{®} or Q7-9120 silicone fluid with a viscosity between 20cst and 12500cst or the product sold under the trade name ST-Cyclomethicone -5NF by the company Dow corning.

Solid fatty substances such as natural or synthetic waxes, fatty acids such as stearic acid, fatty alcohols such as Speziol C18 Pharma sold by the company Cognis and texture agents such as tribehenate, for example Compritol 888 sold by the company Gattefossé or hydrogenated castor oils such as Cutina HR sold by the company Cognis may also be introduced. In this case, those skilled in the art will adjust the heating temperature for the preparation according to the presence or absence of these solids.

For the composition according to the invention, synthetic and/or silicone oils, and more particularly Marcol 152® et la ST-5cyclomethicone -5NF, are preferred.

The hydrophilic phase of the emulsion according to the invention is preferably aqueous and may therefore comprise water. This water may in particular be a floral water such as cornflower water, or a natural mineral water or spring water, for example chosen from Vittel water, water from the Vichy basin, Uriage water, La Roche Posay water, Avène water or Aix-les-Bains water.

Said aqueous phase may be present at a content of between 10% and 90% by weight, relative to the total weight of the composition, preferably between 20% and 80% by weight.

The subject of the present invention is also the composition as described above, as a medicament.

In particular, the invention relates to the use of at least one retinoid compound, benzoyl peroxide and at least one anti-irritant compound described above, or of a composition as described above, in the preparation of a medicament for use in the treatment and/or prevention of dermatological conditions linked to a keratinization disorder relating to cell differentiation and proliferation, in particular for treating common acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and acne medicamentosa.

Preferably, the invention relates to the use of a composition as described above, in the preparation of a medicament for use in preventing and/or treating common acne.

In addition, the invention also relates to the cosmetic use of a composition according to the invention, in the treatment of skin with an acneic tendency, for combating the greasy appearance of the skin or the hair.

A subject of the invention is also a method for preparing a composition as described above. Such a method comprises a step of mixing at least one retinoid compound as defined above, preferably present in a physiologically acceptable medium, with benzoyl peroxide and with at least one anti-irritant compound, said retinoid compounds and benzoyl peroxide being in a dispersed form in said composition.

The introduction of the other optional excipients and additives will be carried out according to the chemical nature of the compounds and the galenical form chosen. For more clarity in the following descriptions of processes, by lipophilic compound, it is meant a substance having an affinity for, tending to combine with, or capable of dissolving in lipids, fat or oils.

By hydrophilic ingredients, it is meant a substance having a strong affinity for water, tending to dissolve in, mix with, or be wetted by water.

The preparation of a composition according to the invention is carried out according to a general process as follows:
a) the retinoid compound is mixed with at least one wetting agent in water, until said retinoid compound is completely dispersed, in order to obtain the active phase 1;
b) the benzoyl peroxide is mixed with at least one wetting agent in water, until said benzoyl peroxide is completely dispersed, in order to obtain the active phase 2;
c) an aqueous phase comprising water, at least one anti-irritant, at least one hydrophilic ingredients is prepared, optionally, add the gelling agent;
d) optionally, for obtaining an emulsion, mix, if necessary heat up, at least one emulsifier, at least one lipophilic compound and optionally solid fatty substances until homogenization, in order to obtain the fatty phase;
e) Optionally, for obtaining a gel-cream, mix if necessary heat up, at least one oil and/or solid fatty substance until homogenization, in order to obtain the fatty phase;
f) the two active phases obtained respectively in a) and b) are mixed in order to obtain one unique active phase;
g) in case of gel or gel-cream, mix the unique active phase obtained in step f) with aqueous phase obtained in step c);
h) optionally, add the gelling agent
i) in case of emulsion, said fatty phase obtained in step d) is mixed with the aqueous active phase obtained in step c) in order to obtain an emulsion;
j) optionally in case of emulsion, the unique active phase obtained in step e) is mixed with emulsion obtained in step i);
k) optionally, in case of gel-cream, the unique ingredient of fatty phase or the fatty phase obtained in step e) is mixed with the phase obtained in step g) or step h) ;
l) if necessary, heat sensitive additives are added;
m) if necessary, a pH adjuster is introduced into the emulsion obtained in step j) or into the gel obtained in step g) or in step h) or into gel-cream obtained in step k) in order to obtain the desired pH;
n) if necessary, water is added to make up the remainder.

According to alternative embodiment, the composition according to the present invention is prepared as follows:
a') steps a) and b)of the general process as described previously are merged in order to obtained a unique step a') which is the mix of at least the retinoid, the benzoyl peroxide and at least one wetting agent with water until complete dispersion of ingredients in order to obtain a unique active phase.

Steps c), d), e), g), h), i), j), k), 1), m) et n) of the previously described process remain unchanged accordingly.

More specifically, a first embodiment of the present invention is the method or the process for preparing a composition according to the invention in the form of a gel, comprising the following steps:
a) mixing at least one retinoid with water and, at least a wetting agent until complete dispersion, in order to obtain the active phase 1;
b) mixing the benzoyl peroxide with water and, at least a wetting agent, until complete dispersion, in order to obtain the active phase 2;
c) preparing an aqueous phase comprising water, at least one anti-irritant, at least one hydrophilic agent, optionally, add the gelling agent;
d) the active phases 1 and 2 respectively obtained in step a) and step b) are mixed in order to obtain a unique active phase;
e) the unique active phase obtained in step d) is mixed with the aqueous phase obtained in step c) and stirring until complete homogenization;
f) optionally, add the gelling agent;
g) if necessary, heat sensitive additives are added;
h) if necessary, a pH adjuster is introduced into the phase obtained in step d) or in step e) or in step f) in order to obtain the desired pH;
i) if necessary, water is added to make up the remainder.

More specifically, according to particular embodiment of the invention, one object of the invention is an alternative process of preparation of the instant invention in a form of a gel, comprising the following steps:
a') steps a) and b)of the general process as described previously are merged in order to obtained a unique step a') which is the mix of at least the retinoid, the benzoyl peroxide and at least one wetting agent with water until complete dispersion of ingredients in order to obtain a unique active phase.

Steps c), d), f), g), h), i) of the previously described process remain unchanged accordingly.

According to another embodiment, the method for preparing the compositions according to the invention in the form of a cream-gel, comprises successively the following steps of:
a) mixing at least one retinoid with water and,at least one wetting agent, until complete dispersion, in order to obtain the active phase 1;
b) mixing the benzoyl peroxide with water and, et least one a wetting agent, until complete dispersion, in order to obtain the active phase 2;
c) preparing an aqueous phase comprising water, at least one anti-irritant and, at least one hydrophilic agent, optionally, add the gelling agent;
d) optionally, mixing at least two lipophilic compounds in order to obtain the fatty phase;
e) the active phases 1 and 2 respectively obtained in step a) and step b) are mixed together in order to obtain a unique active phase;
f) the unique active phase obtained in step d) is mixed with the aqueous phase obtained in c)
g) optionally, add the gelling agent;
h) add the unique ingredient of fatty phase or optionally the fatty phase obtained in step d) in the gel obtained in step f) or in step g) in order to obtain a gel-cream;
i) if necessary, heat sensitive additives are added;
j) if necessary, a pH adjuster is introduced gel-cream obtained in step h) or in step i);
k) if necessary, water is added to make up the remainder.

More specifically, according to particular embodiment of the invention, one object of the invention is an alternative process of preparation of the instant invention in a form of a gel-cream, comprising the following steps:
a') steps a) and b)of the general process as described previously are merged in order to obtained a unique step a') which is the mix of at least the retinoid, the benzoyl peroxide and at least one wetting agent with water until complete dispersion of ingredients in order to obtain a unique active phase.

Steps c), d), e), f), g), h), i) j), k) of the previously described process remain unchanged accordingly.

According to a third embodiment, the method for preparing the compositions according to the invention in the form of an emulsion comprises successively the following steps of:
a) mixing at least one retinoid with water and, at least one wetting agent, until complete dispersion, in order to obtain the active phase 1;
b) mixing the benzoyl peroxide with water and, at least one wetting agent, until complete dispersion, in order to obtain the active phase 2;
c) preparing an aqueous phase comprising water, at least one anti-irritant and, at least one hydrophilic agent ;
d) the active phases 1 and 2 respectively obtained in step a) and step b) are mixed together in order to obtain a unique active phase;
e) mixing at least one emulsifier with at least one lipophilic compound in order to obtain the fatty phase;
f) the fatty phase obtained in step e) is mixed with the aqueous phase obtained in step c) in order to obtain an emulsion.
g) the unique active phase obtained in step d) is mixed with the emulsion obtained in step f)
h) optionally, add the gelling agent;
i) if necessary, heat sensitive additives are added;
j)if necessary, a pH adjuster is introduced in emulsion obtained in step h);
k) if necessary, water is added to make up the remainder.

More specifically, according to particular embodiment of the invention, one object of the invention is an alternative process of preparation of the instant invention in a form of an emulsion, comprising the following steps:
a') steps a) and b)of the general process as described previously are merged in order to obtained a unique step a') which is the mix of at least the retinoid, the benzoyl peroxide and at least one wetting agent with water until complete dispersion of ingredients in order to obtain a unique active phase.

Steps c), d), e), f), g), h), i) j), k) of the previously described process remain unchanged accordingly.

The methods for preparing the compositions according to the invention are mentioned by way of examples, and cannot limit the scope of the present invention.

Finally, another subject of the invention relates to the non-therapeutic cosmetic treatment process for embellishing the skin or its surface appearance, in which a composition according to the invention in a physiologically acceptable medium is applied to the skin and/or its appendages.

The present invention will now be illustrated by means of the following examples.

### EXAMPLES

### Example 1 Formulation of cream type comprising 0,1% adapalene, 2.5% benzoyl peroxide and enoxolone as anti-irritant :

The formula is prepared according to above detailed process of preparation :

| **Constituents** | **content (% m/m)** |
|---|---|
| Benzoyl Peroxide | 2,50 |
| Adapalene | 1.50 |
| Enoxolone | 1.50 |
| Synperonic PE/L44 | 0,20 |
| Sodium Docusate | 0,05 |
| Propylene glycol | 6,00 |
| EDTA | 0,10 |
| Carbopol Ultrez 20 | 0,40 |
| Glycerine | 3,00 |
| Glucamate SSE 20 | 3,50 |
| Glucate SS | 3,50 |
| Cosbiol | 6,00 |
| ST-Cyclomethicone 5 NF | 13,00 |
| Purified water | qsp 100% |
| Triethanolamine | qsp pH 5, 5±0, 5 |

### Example 2 : Formulation cream type comprising 0,3% adapalene, 5% benzoyl peroxide and sodium salt of 18β-glycyrrhetinic acid as anti-irritant :

The formula is prepared according to above detailed process of preparation :

| **Constituents** | **content (% m/m)** |
|---|---|
| Benzoyl Peroxide | 5,00 |
| Adapalene | 0,30 |
| sodium salt of 18β-glycyrrhetinic acid | 1,50 |
| Dipropylene glycol | 5,00 |
| Synperonic PE/L44 | 0,20 |
| Glycerine | 7,00 |
| Xantural 180 | 0,40 |
| Eumulgin B2 PH | 3,00 |
| Arlacel 165FL | 3,00 |
| Speziol C18 Pharma | 2,00 |
| Mygliol 812 N | 7,00 |
| ST-Cyclomethine 5 NF | 6,00 |
| Simulgel 600 PHA | 2,50 |
| purified water | qsp 100% |
| Sodium Hydroxyde | qsp pH 5, 5±0, 5 |

### Example 3 Formulation of lotion type comprising 0.3% adapalene, 1% benzoyl peroxyde and dipotassium salt of 18β-glycyrrhetinic acid as anti-irritant

The formula is prepared according to above detailed process of preparation :

| **Constituents** | **content (% m/m)** |
|---|---|
| Benzoyl Peroxide | 1.00 |
| Adapalene | 0.30 |
| Dipotassium salt of 18β-glycyrrhetinic acid | 1.50 |
| Avicel CL-611 | 1.50 |
| Dipropylene glycol | 3.00 |
| Synperonic PE/L44 | 0.20 |
| Methyl paraben | 0.15 |
| Brij 721 | 3.00 |
| Arlacel 165FL | 3.00 |
| Propyl paraben | 0.05 |
| Perhydrosqualene | 5.00 |
| Cetiol SN PH | 5.00 |
| Simulgel 600PHA | 1.50 |
| Purified water | qsp 100% |
| Triethanolamine | qsp pH 5.5 +/- 0.5 |

### Example 4 : Formulation of gel type comprising adapalene, 2.5% benzoyl peroxide and potassium salt of 18β-glycyrrhetinic acid as anti-irritant:

The formula is prepared according to above detailed process of preparation :

| **Constituents** | **content (% m/m)** |
|---|---|
| Benzoyl Peroxide | 2.50 |
| Adapalene | 0.10 |
| Potassium salt of 18β-glycyrrhetinic acid | 1.50 |
| Propylene glycol | 4,00 |
| Synperonic PE/L44 | 0,20 |
| EDTA | 0,10 |
| Glycerine | 4.00 |
| Sodium Docusate | 0.05 |
| Simulgel 60PHA | 4.00 |
| Purified water | qsp 100% |

### Example 5 Formulation of gel-cream type comprising 0.1% adapalene, 2.5% benzoyl peroxide and potassium salt of 18β-glycyrrhétinic acid as anti-irritant:

The formula is prepared according to above detailed process of preparation :

| **Constituents** | **content (% m/m)** |
|---|---|
| Benzoyl Peroxide | 2.50 |
| Adapalene | 0.10 |
| Potassium salt of 18β-glycyrrhetinic acid | 1.50 |
| Propylene glycol | 6.00 |
| Synperonic PE/L44 | 0.20 |
| Glycerine | 5.00 |
| ST-Cyclométhicone 5NF | 7.00 |
| Simulgel 600PHA | 4.00 |
| Purified water | qsp 100% |

### Example 6: Formulation of gel type comprising 0.3% adapalene, 2.5% benzoyl_peroxide and the monoammonium salt of 180-glycyrrhetinic acid as anti-irritant:

The formula is prepared according to above detailed process of preparation :

| | |
|---|---|
| Constituents | Content (% m/m) |
| Adapalene | 0.30 |
| Monoammonium salt of 18β-glycyrrhetinic acid | 1.50 |
| Benzoyl peroxide | 2.50 |
| Titriplex III | 0.20 |
| Simulgel 600 | 2.00 |
| Propylene glycol | 4.00 |
| Synperonic PE/L62 | 0.20 |
| Phenoxyethanol | 1.00 |
| Purified water | qs 100 |
| Sodium hydroxide 10% m/m | qs pH 5.5 ± 0.5 |

### Example 7: Formulation of gel type comprising 0.3% adapalene, 5% benzoyl peroxide and the disodium succinate salt of 180-glycyrrhetinic acid as anti-irritant:

The formula is prepared according to above detailed process of preparation :

| Constituents | Content (% m/m) |
|---|---|
| Adapalene | 0.30 |
| Disodium succinate salt of 18β-glycyrrhetinic acid | 1.50 |
| Benzoyl peroxide | 5.00 |
| Titriplex III | 0.20 |
| Natrosol 250 HHX Pharm | 2.00 |
| Propylene glycol | 4.00 |
| Synperonic PE/L62 | 0.20 |
| Phenoxyethanol | 1.00 |
| Purified water | qs 100 |

### Example 8: Formulation of emulsion type comprising 0.1% adapalene, 2.5% benzoyl peroxide and the dipotassium salt of 180-glycyrrhetinic acid as anti-irritant:

The formula is prepared according to above detailed process of preparation :

| Constituents | Content (% m/m) |
|---|---|
| Benzoyl peroxide | 2.50 |
| Adapalene | 0.10 |
| Dipotassium salt of 18β-glycyrrhetinic acid | 1.50 |
| Propylene glycol | 2.00 |
| Synperonic PE/L62 | 0.20 |
| HEDTA | 0.10 |
| Nipagin M (optional) | 0.20 |
| Carbopol ultrez 20 | 0.15 |
| Veegum K | 0.30 |
| Glycerol | 3.00 |
| Phenoxyethanol | 1.00 |
| Nipasol M (optional) | 0.20 |
| Glucate SS | 1.00 |
| Glucamate SSE20 | 5.00 |
| Miglyol 812 N | 9.00 |
| Q7-9120 silicone fluid 20 cst | 1.00 |
| Purified water | qs 100 |
| Sodium hydroxide 10% m/m | qs pH 5.5 ± 0.5 |

### Example 9: Formulation of emulsion type comprising 0.3% adapalene, 2.5% benzoyl peroxide and the potassium salt of 180-glycyrrhetinic acid as anti-irritant:

The formula is prepared according to above detailed process of preparation :

| Constituents | Content (% m/m) |
|---|---|
| Benzoyl peroxide | 2.50 |
| Adapalene | 0.30 |
| Potassium salt of 18β-glycyrrhetinic acid | 1.50 |
| Propylene glycol | 2.00 |
| Synperonic PE/L62 | 0.20 |
| HEDTA | 0.10 |
| Nipagin M (optional) | 0.20 |
| Carbopol ultrez 20 | 0.15 |
| Veegum K | 0.30 |
| Glycerol | 3.00 |
| Phenoxyethanol | 1.00 |
| Nipasol M (optional) | 0.20 |
| Glucate SS | 1.00 |
| Glucamate SSE20 | 5.00 |
| Miglyol 812 N | 9.00 |
| Q7-9120 silicone fluid 20 cst | 1.00 |
| Purified water | qs 100 |
| Sodium hydroxide 10% m/m | qs pH 5.5 ± 0.5 |

### Example 10: Formulation of emulsion type comprising 0.1% adapalene, 2.5% benzoyl peroxide and 18β-glycyrrhetinic acid disodium succinate as anti-irritant:

The formula is prepared according to above detailed process of preparation :

| Constituents | Content (% m/m) |
|---|---|
| Benzoyl peroxide | 2.50 |
| Adapalene | 0.10 |
| 18β-Glycyrrhetinic acid disodium succinate | 1.50 |
| Propylene glycol | 4.00 |
| Synperonic PE/L44 | 0.20 |
| HEDTA | 0.10 |
| Nipagin M (optional) | 0.20 |
| Carbopol ETD2020 | 0.20 |
| Stearyl alcohol | 1.00 |
| Phenoxyethanol | 1.00 |
| Nipasol M (optional) | 0.10 |
| Tefose 1500 | 5.00 |
| Miglyol 812 N | 7.00 |
| Purified water | qs 100 |
| Sodium hydroxide 10% m/m | qs pH 5.5 ± 0.5 |

### Example 11: Formulation of cream-gel type comprising 0.3% adapalene, 5% benzoyl peroxide and enoxolone as anti-irritant:

The formula is prepared according to above detailed process of preparation :

| Constituents | Content (% m/m) |
|---|---|
| Benzoyl peroxide | 5.00 |
| Adapalene | 0.30 |
| Enoxolone | 2.00 |
| Propylene glycol | 7.00 |
| Synperonic PE/L44 | 0.20 |
| HEDTA | 0.10 |
| Nipagin M (optional) | 0.20 |
| Glycerol | 5.00 |
| Simulgel 600 | 3.00 |
| Phenoxyethanol | 1.00 |
| Nipasol M (optional) | 0.10 |
| Miglyol 812 | 7.00 |
| Purified water | qs 100 |
| Sodium hydroxide 10% m/m | qs pH 5.5 ± 0.5 |

## Claims

1. Composition for topical application comprising, in a physiologically acceptable medium:
- at least one retinoid compound in dispersed form chosen from all-trans retinoic acid, isotretinoin, motretinide, and naphthoic acid derivatives of formula (I), and salts and esters thereof: 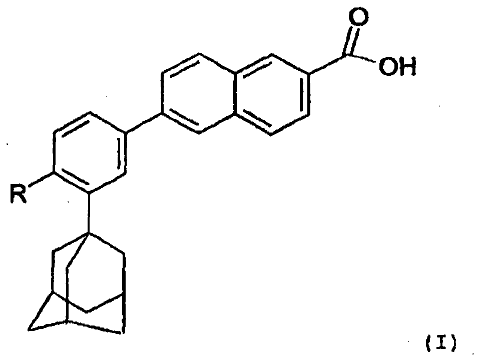 where R represents a hydrogen atom, a hydroxyl radical, a branched or nonbranched alkyl radical containing from 1 to 4 carbon atoms, an alkoxy radical containing from 1 to 10 carbon atoms or a cycloaliphatic radical which is substituted or unsubstituted,
- at least one wetting agent,
- benzoyl peroxide in dispersed form, and
- at least one anti-irritant compound chosen from 18β-glycyrrhetinic acid, the potassium salt of 18β-glycyrrhetinic acid, the sodium salt of 18β-glycyrrhetinic acid, the monoammonium salt of 18β-glycyrrhetinic acid, the disodium succinate salt of 18β-glycyrrhetinic acid, the dipotassium salt of 18β-glycyrrhetinic acid and the monoester of 18β-glycyrrhetinic acid.

2. Composition according to Claim 1, wherein the retinoid compound is a naphthoic acid derivative of formula (I), and salts and esters thereof.

3. Composition according to Claim 2, **characterized in that** the alkyl radical is the methyl, ethyl, propyl or butyl radical; the alkoxy radical is the methoxy, ethoxy, propoxy, butoxy, hexyloxy or decyloxy radical; and the cycloaliphatic radical is the 1-methylcyclohexyl radical or the 1-adamantyl radical.

4. Composition according to either of Claims 2 and 3, **characterized in that** the retinoid compound is chosen from 6-[3-(1-adamantyl)-4-methoxyphenyl]-2-naphthoic acid (adapalene), 6-[3-(1-adamantyl)-4-hydroxyphenyl]-2-naphthoic acid, 6-[3-(1-adamantyl)-4-decyloxyphenyl]-2-naphthoic acid and 6-[3-(1-adamantyl)-4-hexyloxyphenyl]-2-naphthoic acid, salts and esters thereof.

5. Composition according to Claim 4, **characterized in that** the retinoid compound is adapalene, its salts and esters thereof.

6. Composition according to any one of the preceding claims, **characterized in that** the concentration of retinoid compound is between 0.001% and 10%, preferentially between 0.01% and 5%, preferably between 0.01% and 1%, more preferentially between 0.01% and 0.5%, and preferentially between 0.1% and 0.3% by weight of the total weight of the composition.

7. Composition according to Claim 6, **characterized in that** the concentration of retinoid compound is equal to 0.1%.

8. Composition according to Claim 6, **characterized in that** the concentration of retinoid compound is equal to 0.3%.

9. Composition according to any one of the preceding claims, **characterized in that** the concentration of anti-irritant compound is between 0.01% and 10%, preferentially between 0.1% and 7%.

10. Composition according to any one of the preceding claims, **characterized in that** it comprises between 0.0001% and 20% of benzoyl peroxide, preferentially between 0.025% and 10%, even more preferentially between 2.5% and 5%.

11. Composition according to Claim 1, **characterized in that** it is in the form of aqueous, aqueous-alcoholic or oily dispersions, dispersions of the lotion type, aqueous, anhydrous or lipophilic gels, emulsions of liquid or semi-liquid consistency of the milk type, obtained by dispersion of a fatty phase in an aqueous phase (O/W) or vice versa (W/O), or suspensions or emulsions of soft, semi-liquid or solid consistency of the cream, cream-gel, foam or ointment type, or microemulsions, microcapsules, microparticles or vesicular dispersions of ionic and/or nonionic type, in the form of sprays, or in the form of dermal devices such as patches.

12. Composition according to either of Claims 1 and 11, **characterized in that** it is in the form of a gel, a cream-gel or an emulsion.

13. Composition according to any one of the preceding claims, for use as a medicament.

14. Cosmetic use of a composition according to any one of Claims 1 to 12, in the treatment of skin with an acneic tendency or for combating the greasy appearance of the skin.

15. Method for preparing a composition according to Claims 1 to 13, **characterized in that** it comprises a step of mixing at least one retinoid compound with benzoyl peroxide and with at least one anti-irritant compound.

16. Use of a therapeutical effective amount of a composition according to claims 1 to 12 for the preparation of a medicament for treating and/or preventing and/or inhibiting dermatological conditions linked to a keratinization disorder relating to cell differentiation and proliferation, in particular for treating common acne, comedonic acne, papulopustular acne, papulocomedonic acne, nodulocystic acne, acne conglobata, cheloid acne of the nape and/or the neck, recurrent miliary acne, necrotic acne, neonatal acne, occupational acne, acne rosacea, senile acne, solar acne and acne medicamentosa.

17. Nontherapeutic cosmetic treatment process for embellishing the skin or its surface appearance, in which a composition according to one of Claims 1 to 12 is applied to the skin and/or its integument annexes.

## Patentansprüche

1. Zusammensetzung für die topische Anwendung, umfassend in einem physiologisch verträglichen Medium:
- wenigstens eine Retinoidverbindung in dispergierter Form, ausgewählt aus all-trans-Retinolsäure, Isotretinoin, Motretinid und Naphthoesäurederivaten der Formel (I) sowie Salzen und Estern davon: 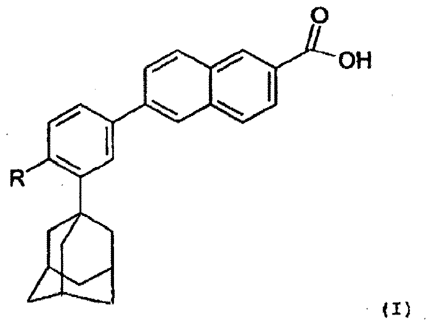 wobei R ein Wasserstoffatom, einen Hydroxyrest, einen verzweigten oder nichtverzweigten Alkylrest mit 1 bis 4 Kohlenstoffatomen, einen Alkoxyrest mit 1 bis 10 Kohlenstoffatomen oder einen cycloaliphatischen Rest, der substituiert oder nichtsubstituiert ist, darstellt,
- wenigstens ein Netzmittel,
- Benzoylperoxid in dispergierter Form und
- wenigstens eine antiirritative Verbindung ausgewählt aus 18β-Glycyrrhetinsäure, dem Kaliumsalz von 18β-Glycyrrhetinsäure, dem Natriumsalz von 18β-Glycyrrhetinsäure, dem Monoammoniumsalz von 18β-Glycyrrhetinsäure, dem Dinatriumsuccinatsalz von 18β-Glycyrrhetinsäure, dem Dikaliumsalz von 18β-Glycyrrhetinsäure und dem Monoester von 18β-Glycyrrhetinsäure.

2. Zusammensetzung gemäß Anspruch 1, wobei die Retinoidverbindung ein Naphthoesäurederivat der Formel (I) ist, und Ester und Salze davon.

3. Zusammensetzung gemäß Anspruch 2, **dadurch gekennzeichnet, dass** der Alkylrest der Methyl-, Ethyl-, Propyl- oder Butylrest ist; der Alkoxyrest der Methoxy-, Ethoxy-, Propoxy-, Butoxy-, Hexyloxy- oder Decyloxyrest ist; und der cycloaliphatische Rest der 1-Methylcyclohexylrest oder der 1-Adamantylrest ist.

4. Zusammensetzung gemäß einem der Ansprüche 2 und 3, **dadurch gekennzeichnet, dass** die Retinoidverbindung ausgewählt ist aus 6-[3-(1-Adamantyl)-4-methoxyphenyl]-2-naphthoesäure (Adapalen), 6-[3-(1-Adamantyl)-4-hydroxyphenyl]-2-naphthoesäure, 6-[3-(1-Adamantyl)-4-decyloxyphenyl]-2-naphthoesäure und 6-[3-(1-Adamantyl)-4-hexyloxyphenyl]-2-naphthoesäure, Salzen und Estern davon.

5. Zusammensetzung gemäß Anspruch 4, **dadurch gekennzeichnet, dass** die Retinoidverbindung Adapalen ist, und Salze und Ester davon.

6. Zusammensetzung gemäß einem der vorstehenden Ansprüche, **dadurch gekennzeichnet, dass** die Konzentration der Retinoidverbindung zwischen 0,001 % und 10 % beträgt, vorzugsweise zwischen 0,01 % und 5 %, bevorzugt zwischen 0,01 % und 1 %, bevorzugter zwischen 0,01 % und 0,5 % und vorzugsweise zwischen 0,1 % und 0,3 % nach Gewicht des Gesamtgewichts der Zusammensetzung.

7. Zusammensetzung gemäß Anspruch 6, **dadurch gekennzeichnet, dass** die Konzentration der Retinoidverbindung 0,1 % beträgt.

8. Zusammensetzung gemäß Anspruch 6, **dadurch gekennzeichnet, dass** die Konzentration der Retinoidverbindung 0,3 % beträgt.

9. Zusammensetzung gemäß einem der vorstehenden Ansprüche, **dadurch gekennzeichnet, dass** die Konzentration der antiirritativen Verbindung zwischen 0,01 % und 10 % beträgt, vorzugsweise zwischen 0,1 % und 7 %.

10. Zusammensetzung gemäß einem der vorstehenden Ansprüche, **dadurch gekennzeichnet, dass** sie zwischen 0,0001 % und 20 % an Benzoylperoxid umfasst, vorzugsweise zwischen 0,025 % und 10 %, noch bevorzugter zwischen 2,5 % und 5 %.

11. Zusammensetzung gemäß Anspruch 1, **dadurch gekennzeichnet, dass** sie in der Form von wässrigen, wässrig-alkoholischen oder öligen Dispersionen, Dispersionen vom Lotionstyp, wässrigen, wasserfreien oder lipophilen Gelen, Emulsionen mit flüssiger oder halbflüssiger Konsistenz vom Milchtyp, erhalten durch Dispergieren einer Fettphase in einer wässrigen Phase (O/W) oder umgekehrt (W/O), oder Suspensionen oder Emulsionen mit weicher, halbflüssiger oder fester Konsistenz vom Creme-, Creme-Gel-, Schaum- oder Salbentyp, oder Mikroemulsionen, Mikrokapseln, Mikropartikel oder vesikuläre Dispersionen vom ionischen und/oder nichtionischen Typ, in der Form von Sprays oder in der Form von dermalen Einheiten, wie z. B. Pflastern, vorliegt.

12. Zusammensetzung gemäß einem der Ansprüche 1 und 11, **dadurch gekennzeichnet, dass** sie in der Form eines Gels, eines Creme-Gels oder einer Emulsion vorliegt.

13. Zusammensetzung gemäß einem der vorstehenden Ansprüche zur Anwendung als Medikament.

14. Kosmetische Verwendung einer Zusammensetzung gemäß einem der Ansprüche 1 bis 12 bei der Behandlung von Haut mit Akneneigung oder zum Bekämpfen von fettigem Aussehen der Haut.

15. Verfahren zum Herstellen einer Zusammensetzung gemäß Ansprüchen 1 bis 13, **dadurch gekennzeichnet, dass** sie einen Schritt des Mischens von wenigstens einer Retinoidverbindung mit Benzoylperoxid und mit wenigstens einer antiirritativen Verbindung umfasst.

16. Verwendung einer therapeutisch wirksamen Menge einer Zusammensetzung gemäß Ansprüchen 1 bis 12 für die Herstellung eines Medikaments zum Behandeln und/oder Verhindern und/oder Hemmen von dermatologischen Zuständen, die mit einer Keratinisierungsstörung verbunden sind, die Zelldifferenzierung und -proliferation betrifft, insbesondere zum Behandeln von gewöhnlicher Akne, Komedonen-Akne, papulopustulöser Akne, Papulokodemonen-Akne, nodulozystischer Akne, Acne conglobata, cheloider Akne von Nacken und/oder Hals, rezidivierender miliarer Akne, nekrotischer Akne, neonataler Akne, berufsbedingter Akne, Acne rosacea, seniler Akne, Sonnenakne und Acne medicamentosa.

17. Nichttherapeutisches kosmetisches Behandlungsverfahren zum Verschönern der Haut oder ihres Oberflächenaussehens, wobei eine Zusammensetzung gemäß einem der Ansprüche 1 bis 12 auf die Haut und/oder ihre Hautanhänge aufgebracht wird.

## Revendications

1. Composition pour application topique comprenant, dans un milieu physiologiquement acceptable :
- au moins un composé rétinoïde sous forme dispersée choisi parmi l'acide rétinoïque tout-trans, l'isotrétinoïne, le motrétinide, et les dérivés d'acide naphtoïque de formule (I), et les sels et esters de ceux-ci : 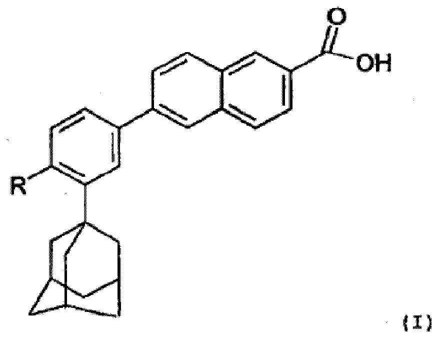 où R représente un atome d'hydrogène, un radical hydroxyle, un radical alkyle ramifié ou non ramifié contenant de 1 à 4 atomes de carbone, un radical alcoxy contenant de 1 à 10 atomes de carbone ou un radical cycloaliphatique substitué ou non substitué,
- au moins un agent mouillant,
- du peroxyde de benzoyle sous forme dispersée, et
- au moins un composé anti-irritant choisi parmi l'acide 18β-glycyrrhétinique, le sel de potassium de l'acide 18β-glycyrrhétinique, le sel de sodium de l'acide 18β-glycyrrhétinique, le sel de mono-ammonium de l'acide 18β-glycyrrhétinique, le sel de succinate disodique de l'acide 18β-glycyrrhétinique, le sel dipotassique de l'acide 18β-glycyrrhétinique et le monoester de l'acide 18β-glycyrrhétinique.

2. Composition selon la revendication 1, dans laquelle le composé rétinoïde est un dérivé d'acide naphtoïque de formule (I), et des sels et des esters de celui-ci.

3. Composition selon la revendication 2, **caractérisée en ce que** le radical alkyle est le radical méthyle, éthyle, propyle ou butyle ; le radical alcoxy est le radical méthoxy, éthoxy, propoxy, butoxy, hexyloxy ou décyloxy ; et le radical cycloaliphatique est le radical 1-méthylcyclohexyle ou le radical 1-adamantyle.

4. Composition selon l'une des revendications 2 et 3, **caractérisée en ce que** le composé rétinoïde est choisi parmi l'acide 6-[3-(1-adamantyl)-4-méthoxyphényl]-2-naphtoïque (adapalène), l'acide 6-[3-(1-adamantyl)-4-hydroxyphényl]-2-naphtoïque, l'acide 6-[3-(1-adamantyl)-4-décyloxyphényl]-2-naphtoïque et l'acide 6-[3-(1-adamantyl)-4-hexyloxyphényl)-2-naphtoïque, et des sels et des esters de ceux-ci.

5. Composition selon la revendication 4, **caractérisée en ce que** le composé rétinoïde est l'adapalène, ses sels et des esters de celui-ci.

6. Composition selon l'une quelconque des revendications précédentes, **caractérisée en ce que** la concentration de composé rétinoïde est comprise entre 0,001 % et 10 %, préférablement entre 0,01 % et 5 %, de préférence entre 0,01 % et 1 %, plus préférablement entre 0,01 % et 0,5 %, et préférablement entre 0,1 % et 0,3 % en poids du poids total de la composition.

7. Composition selon la revendication 6, **caractérisée en ce que** la concentration de composé rétinoïde est égale à 0,1 %.

8. Composition selon la revendication 6, **caractérisée en ce que** la concentration de composé rétinoïde est égale à 0,3 %.

9. Composition selon l'une quelconque des revendications précédentes, **caractérisée en ce que** la concentration de composé anti-irritant est comprise entre 0,01 % et 10 %, de préférence entre 0,1 % et 7 %.

10. Composition selon l'une quelconque des revendications précédentes, **caractérisée en ce qu'**elle comprend entre 0,0001 % et 20 % de peroxyde de benzoyle, de préférence entre 0,025 % et 10 %, encore plus préférablement entre 2,5 % et 5 %.

11. Composition selon la revendication 1, **caractérisée en ce qu'**elle est sous la forme de dispersions aqueuses, hydro-alcooliques ou huileuses, de dispersions de type lotion, de gels aqueux, anhydres ou lipophiles, d'émulsions de consistance liquide ou semi-liquide de type lait, obtenues par dispersion d'une phase grasse dans une phase aqueuse (H/E) ou inversement (E/H), ou de suspensions ou d'émulsions de consistance molle, semi-liquide ou solide de type crème, gel-crème, mousse ou pommade, ou de micro-émulsions, de microcapsules, de microparticules ou de dispersions vésiculaires de type ionique et/ou non ionique, sous la forme d'aérosols, ou sous la forme de dispositifs dermiques tels que des patchs.

12. Composition selon l'une des revendications 1 et 11, **caractérisée en ce qu'**elle est sous la forme d'un gel, d'un gel-crème ou d'une émulsion.

13. Composition selon l'une quelconque des revendications précédentes, pour son utilisation en tant que médicament.

14. Utilisation cosmétique d'une composition selon l'une quelconque des revendications 1 à 12, dans le traitement d'une peau ayant une tendance acnéique ou pour lutter contre l'aspect gras de la peau.

15. Procédé de préparation d'une composition selon les revendications 1 à 13, **caractérisé en ce qu'**il comprend une étape de mélange d'au moins un composé rétinoïde avec du peroxyde de benzoyle et avec au moins un composé anti-irritant.

16. Utilisation d'une quantité thérapeutique efficace d'une composition selon les revendications 1 à 12 pour la préparation d'un médicament pour traiter et/ou prévenir et/ou inhiber les affections dermatologiques liées à un trouble de la kératinisation associé à la différenciation et la prolifération cellulaire, en particulier pour traiter l'acné vulgaire, l'acné comédonienne, l'acné papulo-pustuleuse, l'acné papulo-comédonienne, l'acné nodulo-kystique, l'acné conglobata, l'acné chéloïdienne de la nuque et/ou du cou, l'acné miliaire récidivante, l'acné nécrotique, l'acné néonatale, l'acné professionnelle, l'acné rosacée, l'acné sénile, l'acné solaire et l'acné médicamenteuse.

17. Procédé de traitement cosmétique non thérapeutique pour embellir la peau ou son aspect de surface, dans lequel une composition selon l'une des revendications 1 à 12 est appliquée sur la peau et/ou ses annexes tégumentaires.
